# EUROPÄISCHE PATENTANMELDUNG

(11) **EP 2 404 625 A2**
(43) Veröffentlichungstag der Anmeldung: **11.01.2012**
(21) Anmeldenummer: 11005383.2
(22) Anmeldetag: 01.07.2011
(51) Int. Cl.: A61L 29/02, A61L 29/16

(54) **Katheter zur teilweisen intrakorporaten Verwendung**

(30) Priorität: 06.07.2010 DE 102010017765
(71) Anmelder: KME Germany AG & Co. KG, 49074 Osnabrück (DE)
(72) Erfinder: Mommertz, Josef, 52477 Alsdorf (DE); Sbrana, Armando, 55100 Lucca (IT); Schreiner, Stephan, 49086 Osnabrück (DE); Warnecke, Holger, Dr., 52066 Aachen (DE)
(74) Vertreter: Pietrzykowski, Anja

(57) **Zusammenfassung**

Katheter (1a) mit einem zur teilweise intrakorporalen Verwendung vorgesehenen Katheterabschnitt (2). Erfindungsgemäß ist der Katheterabschnitt (2) durch eine Platte (4a) hindurch geführt, wobei zumindest die Oberfläche der Platte (4a) eine antimikrobiell wirksame Metall-Legierung aufweist. Der Katheterabschnitt (2) ist zumindest bereichsweise von einem Röhrchen (6) umgeben, wobei zumindest die Oberfläche des Röhrchens (6) eine anitmikrobielle Metall-Legierung aufweist. Die Platte (4a) ist im eingeführten Zustand des Katheterabschnitts (2) auf einem umliegenden Hautbereich (5) durch ein Mittel zur Lagefixierung extrakorporal festgelegt. Die Metall-Legierung ist bevorzugt eine Cu-Legierung, welche mehr als 60 Gew.-% Kupfer aufweist.

## Beschreibung

Die Erfindung betrifft einen Katheter mit den Merkmalen im Oberbegriff des Patentanspruchs 1.

Katheter werden in der medizinischen Anwendung für eine Vielzahl diagnostischer oder therapeutischer Zwecke eingesetzt. In Form von Röhrchen oder Schläuchen dienen sie der teilweise intrakorporalen Verwendung, wobei der hierfür vorgesehene Katheterabschnitt in einen mit biologischem Gewebe umgebenen Lumen eingeführt wird. Die so katheterisierten Hohlorgane oder Körperhöhlen können hierüber sondiert, entleert, gefüllt oder gespült werden.

Als zumeist biegsames, schlauchförmiges Instrument verbleibt der Katheter temporär oder dauerhaft in seiner intrakorporalen Lage. Sowohl bei der Verwendung in vorhandenen Körperöffnungen als auch bei subkutaner Anordnung besteht Infektionsgefahr, weil Keime außen am Katheter entlang in den Körper eindringen können. Hierbei gilt es die insbesondere durch Biofilmbildung auf der äußeren Oberfläche des Katheters sowie durch Keimentwicklung auf der Haut im penetrierten Bereich hervorgerufenen Infektionen durch extraluminale Keimaszension zu reduzieren.

Sterile Bedingungen sowie eine ausreichende Desinfektion und der Einsatz beispielsweise von antibiotischen Salben genügen diesen Anforderungen nur temporär. Insgesamt richten sich die Erfordemisse an einen lang anhaltenden sterilisierenden Effekt am Insertionsort, welcher dauerhaft vor möglichen Infektionen schützt

Aus der DE 32 28 849 A1 ist ein in den Körper einzuführendes medizinisches Gerät, insbesondere einen Hamröhrenkatheter, bekannt, welcher mit einem Überzug aus mindestens einer mikrobentötenden Metallionen abgebenden Substanz in Form eines Metalls ausgestattet ist. Die Abgabe von Metallionen geschieht hierbei vorzugsweise aus Gold, Silber oder Kupfer. Die Schichtstärke ist an die jeweilige Anforderung sowie Anwendung angepasst.

Durch den metallischen Überzug wird eine antimikrobielle Wirkung erzielt. Bei der Verwendung bevorzugt hochflexibler Materialien, beispielsweise Gummi oder Silikon, ergeben sich hohe Anforderungen an die Haftung des metallischen Überzugs. Um die elastischen Eigenschaften beizubehalten, kann lediglich eine dünne metallische Schicht aufgetragen werden. Entsprechend kurz fällt die mögliche Nutzungsdauer aus. da der metallische Überzug insbesondere innerhalb durchströmter Gefäße schnell verschleißt. Somit lässt die dauerhafte Kombination aus hochflexiblen Kathetermateriallen und einer dauerhaft antimikrobiellen metallischen Beschichtungen noch Raum für Verbesserungen.

Der vorliegenden Erfindung liegt die Aufgabe zu Grunde, einen Katheter zur teilweise intrakorporalen Verwendung dahingehend zu verbessern, dass trotz des Einsatzes hochflexibler Katheterwerkstoffe eine wirksame Barriere zur Prävention katheterassoziierter Infektionen geschaffen wird.

Die Lösung dieser Aufgabe besteht nach der Erfindung in einem Katheter gemäß den Merkmalen von Patentanspruch 1.

Hiernach ist der zur teilweise intrakorporalen Verwendung vorgesehene Katheterabschnitt durch eine Platte hindurchgeführt, wobei zumindest die Oberfläche der Platte eine antimikrobiell wirksame Metall-Legierung aufweist. Die Platte kann hierfür nur zum Teil oder komplett aus einer Metall-Legierung gebildet sein. Die Platte selbst verbleibt im eingeführten Zustand des Katheterabschnitts extrakorporal. Der besondere Vorteil liegt somit in der Lage der Platte außerhalb des Körpers, wodurch die Größe der Platte nicht durch die Größe des Lumens beschränkt ist. Der intrakorporale Katherterabschnitt kann uneingeschränkt hochflexibel ausgestaltet sein. Die Platte trägt zu keinerlei Querschnittsvergrößerung des einzuführenden Katheterabschnitts bei.

Beim Einsatz des Katheters in stark abschwemmenden Bereichen, beispielsweise innerhalb eines Blutstroms, treten keine Abnutzungserscheinungen der antimikrobiell wirksamen Metall-Legierungen auf. Dadurch werden die antimikrobiellen Eigenschaften dauerhaft beibehalten.

Die Metallplatte kann beispielsweise abgerundete Ecken aufweisen, um Mikroläsionen der Haut zu vermeiden. Für die Durchführung des Katheterabschnitts weist die Platte eine Öffnung in Form eines Loches auf. Je nach Ausgestaltung kann die Platte auch von ihrem Rand aus geschlitzt sein, um diese nachträglich auf den bereits verlegten Katheterabschnitt seitlich aufzuschieben. Bei der Verwendung zweier geschlitzter Platten können diese jeweils von der Gegenseite aus aufgeschoben werden, um den Katheter vollständig zu umgreifen.

Vorteilhafte Weiterbildungen des grundsätzlichen Erfindungsgedankens sind Gegenstand der abhängigen Ansprüche 2 bis 10.

Der einzuführende Katheterabschnitt weist zumeist einen runden Durchmesser auf. Ferner weist die Platte eine Breite auf, wobei der Durchmesser des Katheterabschnitts und die Breite der Platte mindestens ein Verhältnis von 1 : 2 zueinander aufweisen. Die Platte kann als Begrenzung der einzuführenden Länge des Katheterabschnitts dienen. Die auf eine Ebene projizierte Grundform der Platte kann in Anpassung an die individuellen und auch anatomischen Anforderungen prinzipiell beliebig sein, beispielsweise ist sie kreisrund oder rechteckig. Die Breite der Platte bezieht sich hierbei auf den Abstand zwischen zwei Extremalwerten der Ebenenprojektion der Platte.

Weiterhin weist die Platte eine Dicke auf, wobei die Dicke und die Breite der Platte mindestens ein Verhältnis von 1 : 5 zueinander aufweisen. Über die Dicke der Platte kann deren mögliche Verformbarkeit eingestellt werden.

Die Platte kann einen Grundkörper beinhalten, welcher eine antimikrobiell wirksame Metall-Legierung seiner Oberfläche aufweist. Die Dicke der Metall-Legierung gegenüber der Dicke der Platte richtet sich nach den entsprechenden Anforderungen. Grundsätzlich kann die Platte auch aus einem Vollmaterial hergestellt sein, wodurch die Dicke der Metall-Legierung der Dicke der Platte entspricht.

Die Erfindung sieht vor, dass die Platte flach oder kalottenförmig ausgebildet ist. Grundsätzlich sind auch weitere Formgebungen denkbar, die an den jeweiligen Einsatzweck angepasst sind. Die räumliche Formgebung der Platte folgt hierbei den anatomischen Gegebenheiten, welche sich den extrakorporalen Formgebungen anpasst.

In vorteilhafter Weise ist der Katheterabschnitt zumindest bereichsweise von einem Röhrchen umgeben. Die Oberfläche des Röhrchens weist hierbei eine antimikrobielle Metall-Legierung auf. Grundsätzlich kann das Röhrchen auch aus einem Vollmaterial hergestellt sein. Der Innendurchmesser des Röhrchens ist so bemessen, dass das Röhrchen eng an dem Katheterabschnitt anliegt. Auch wenn das Röhrchen und die Platte voneinander beabstandet sein können, liegen diese in vorteilhafter Weise eng beieinander und stehen in Kontakt. Je nach Ausgestaltung weisen das Röhrchen und die Platte einen durchgehenden Kontakt zueinander auf, wobei die jeweiligen Oberflächen sich mit ihrer Metall-Legierung berühren. In einer Variante ist die Platte mit dem Röhrchen verbunden. Es ist auch denkbar, dass die Platte an ihrer Öffnung für den Katheter eine kragenartige Ausstülpung aufweist.

Die antimikrobielle Wirkung der Metall-Legierung beruht darauf, dass Metallionen über die Oberfläche abgegeben werden. Die Metallionen führen zu einer Schädigung der Mikroorganismen, wodurch deren Absterben herbeigeführt wird. Die Metall-Legierung kann hierfür aus diversen Metallen gebildet sein, beispielsweise aus Silber, Messing oder Bronze. Erfindungsgemäß ist die Metall-Legierung eine Cu-Legierung, welche mehr als 60 Gew.-% Kupfer aufweist. Durch oxidierend wirkende Substanzen, wie beispielsweise Luftsauerstoff, Feuchtigkeit oder Körperschweiß ist die Metall-Legierung an ihrer Oberfläche in der Lage, elektrisch neutrale Metallatome in Metallionen umzuwandeln. Mit steigendem Kupfergehalt erhöht sich die antimikrobielle Wirkung der Metall-Legierung.

Da die Platte für den direkten Hautkontakt vorgesehen ist, sollte diese ein möglichst geringes Allergiepotential aufweisen. Für eine allergenfreie Zusammensetzung weist die Metall-Legierung daher jeweils maximal 0,01 Gew.-% an Nickel und/oder Blei auf.

Es ist vorgesehen, dass die Platte und/oder das Röhrchen gegenüber dem Katheterabschnitt relativ verschieblich sind/ist. Durch die Verschieblichkeit des Röhrchens und/oder der Platte erhöht sich die individuelle Verwendbarkeit des Katheters, da die Lage und die Länge des intrakorporal zu verwendenden Katheterabschnitts entsprechend einstellbar sind. Die Platte ist auch in ihrer Lage relativ zur Längsrichtung des Katheterabschnitts neigbar, wodurch sie den anatomischen Gegebenheiten anpassbar ist.

Die Platte ist im eingeführten Zustand des Katheterabschnitts auf einem umliegenden Hautbereich durch ein Mittel zur Lagefixierung extrakorporal festgelegt. Hierdurch wird sichergestellt, dass trotzt Bewegung des Katheters ein permanenter Hautkontakt der Platte erreicht wird.

Darüber hinaus ist vorgesehen, dass das Mittel zur Lagefixierung einzelne Fäden aus einer antimikrobiellen Metall-Legierung aufweist. Um die antimikrobielle Wirkweise weiterhin zu erhöhen, sind/ist die Platte und/oder das Röhrchen mit dem Mittel zur Lagefixierung zumindest bereichsweise umwickelt. Das Mittel zur Lagefixierung kann beispielsweise Verbandmull sein, welches zur Polsterung und Fixierung des Katheters dient. Auch ein selbstklebendes Tape oder Pflaster kann als Mittel zur Lagefixierung eingesetzt werden.

Die Metall-Legierung der Platte steht im ständigen Hautkontakt. Die extrakorporale Anordnung ist, anders als bei einer intrakorporalen Verwendung, vor Abnutzung, beispielsweise durch Abwaschungen, geschützt. Während der gesamten Nutzungsdauer des Katheters reduziert das Röhrchen eine mögliche Biofilmbildung umfangsseitig des Katheterabschnitts. Durch die Reduzierung des Biofilms wird das Infektionsrisiko durch Zutritt von Mikroorganismen entlang des Katheters in den Patientenkörper reduziert. Die auf der Haut aufliegende Platte entfaltet bereits außerhalb des zu katheterisierenden Lumens ihre antimikrobielle Wirkweise, wodurch Mikroorganismen bereits weit vor der möglichen Eintrittsstelle in den Körper gestoppt werden.

Die Erfindung wird nachfolgend anhand eines in den Zeichnungen schematisch dargestellten Ausführungsbeispiels näher beschrieben. Es zeigen:
- Figur 1: einen erfindungsgemäßen Katheter in geschnittener Darstellungsweise;
- Figur 2: einen Katheterteil in einer Aufsicht;
- Figur 3: einen Katheter gemäß der Darstellungsweise von Figur 1 in einer Variante sowie
- Figur 4: einen Katheterteil der Figur 3 in einer Aufsicht.

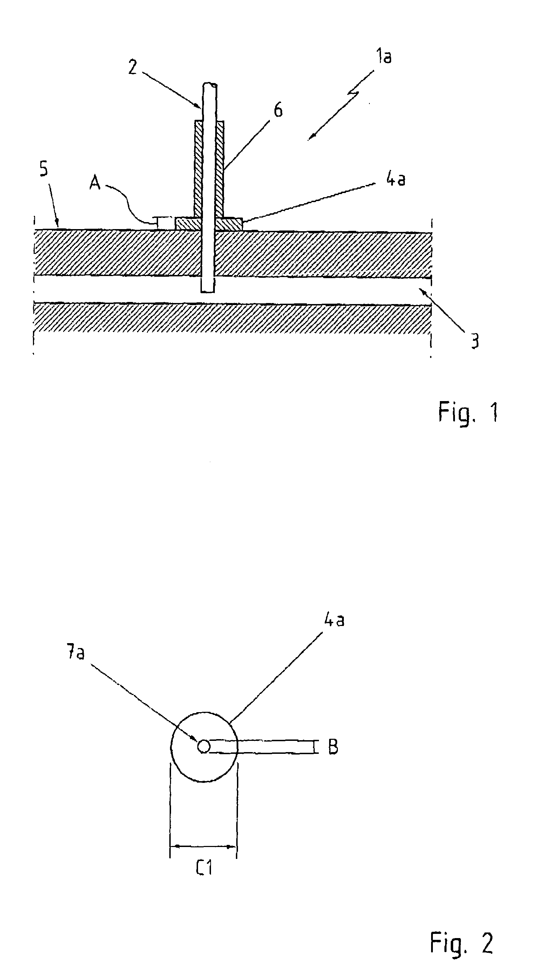

Figur 1 zeigt einen erfindungsgemäßen Katheter 1a in seiner teilweise intrakorporalen Verwendung. Hierfür ist der Katheter 1a mit seinem Endbereich eines Katheterabschnitts 2 in ein subkutanes Gefäß 3 eingeführt. Der Katheterabschnitt 2 ist durch eine Platte 4a hindurchgeführt, welche einen flächigen Kontakt mit einem oberhalb des Gefäßes 3 liegenden Hautbereich 5 aufweist. Die Platte 4a ist in ihrer Lage auf dem Hautbereich 5 mit einem nicht näher dargestellten Mittel zur Lagefixierung extrakorporal festgelegt. Auf einer dem Hautbereich 5 abgewandten Seite der Platte 4a weist der Katheterabschnitt 2 ein Röhrchen 6 auf, welches den Katheterabschnitt 2 bereichsweise umgibt. Sowohl die Platte 4a als auch das Röhrchen 6 weisen eine nicht näher dargestellte Oberfläche aus einer antimikrobiell wirksamen Metall-Legierung auf. Die Platte 4a weist hierbei eine flache Formgebung auf, welche bündig auf dem Hautbereich 5 aufliegt. Die Platte 4a weist hierbei eine Dicke A auf.

Figur 2 zeigt die bereits in Figur 1 dargestellte Platte 4a in einer Aufsicht. Hierbei ist erkenntlich, dass die Platte 4a eine kreisrunde Formgebung besitzt. Im Zentrum der Platte 4a ist eine kreisrunde Öffnung 7a angeordnet, durch welche der Katheterabschnitt 2 des Katheters 1a hindurchgeführt ist. Wie den Figuren 1 und 2 zu entnehmen ist, liegen sowohl die Platte 4a als auch das Röhrchen 6 eng an dem Außenumfang des Katheterabschnitts 2 an. Der Katheterabschnitt 2 weist hierbei einen Durchmesser B auf, während die Platte 4a eine Breite C1 aufweist. Der Durchmesser B und die Breite C1 stehen in einem Verhältnis von 1 : 5 zueinander. Die Dicke A der Platte 4a und der Durchmesser B des Katheterabschnitts 2 weisen ebenfalls ein Verhältnis von 1 : 5 zueinander auf.

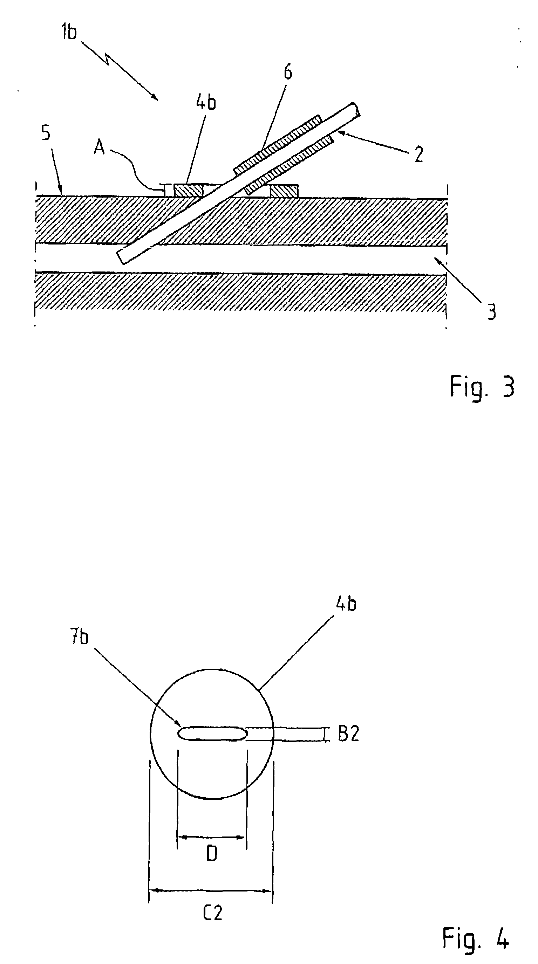

Figur 3 zeigt eine Variante des in Figur 1 dargestellten Katheters 1a. In Form eines Katheters 1b ist dieser in einem flachen Winkel zum Hautbereich 5 in das subkutane Gefäß 3 eingeführt. Der Katheterabschnitt 2 ist hierbei durch eine Platte 4b hindurchgeführt, welche ebenfalls einen flächigen Kontakt mit dem Hautbereich 5 aufweist. Analog der Figur 1 weist auch hier der Katheterabschnitt 2 das Röhrchen 6 auf, welches auf einer dem Hautbereich 5 abgewandten Seite der Platte 4b angeordnet ist.

Figur 4 zeigt die bereits in Figur 3 in geschnittener Darstellungsweise aufgezeigte Platte 4b in einer Aufsicht. Der in Figur 3 in einem flachen Winkel in das Gefäß 3 eingeführte Katheterabschnitt 2 verlangt eine spezielle Geometrie einer in der Platte 4b eingebrachten Öffnung 7b. Die Öffnung 7b weist hierbei einen länglichen Verlauf in Form eines Schlitzes auf, dessen Anfangs- sowie Endbereich gerundet ist. Während die Platte 4b eine Breite C2 aufweist, zeigt die Öffnung 7b auf Grund Ihrer geschlitzten Ausformung eine Länge D. Die Länge D und die Breite C2 stehen in einem Verhältnis von 1 : 1,8 zueinander.

Figur 5 zeigt eine Variante der in den Figuren 2 und 4 in einer Aufsicht dargestellten Platten 4a, 4b. Diese weist in Form einer Platte 4c eine Öffnung 7c auf, welche in Form eines Schlitzes ausgebildet ist. Die Öffnung 7c erstreckt sich hierbei vom Randbereich der Platte 4c aus in dessen Zentrum. Durch die Öffnung 7c erhält die Platte 4c im Wesentlichen eine U-Form. Die Tiefe der Öffnung 7c entspricht dabei zwei Drittel einer Breite C3 der Platte 4c.

Figur 6 zeigt die bereits in Figur 5 dargestellte Platte 4c in Kombination mit einer weiteren Platte 4c. Diese sind mit ihren jeweiligen Öffnungen 7c so gegeneinander geschoben, dass die Öffnung 7c der in der Darstellung oberen Platte 4c durch einen Bereich 8 der unteren Platte 4c zu zwei Dritteln bedeckt ist. Hierdurch bilden die Platten 4c eine gemeinsame Öffnung 9, welche den Katheterabschnitt 2 umgreift.

### Bezugszeichen:

- 1a -: Katheter
- 1b -: Katheter
- 2 -: Katheterabschnitt
- 3 -: Gefäß
- 4a -: Platte
- 4b -: Platte
- 4c -: Platte
- 5 -: Hautbereich
- 6 -: Röhrchen
- 7a -: Öffnung in 4a
- 7b -: Öffnung in 4b
- 7c -: Öffnung in 4c
- 8 -: Bereich von 4c
- 9 -: Öffnung zwischen 4c

- A -: Dicke von 4a, 4b, 4c
- B -: Durchmesser von 2
- C1 -: Breite von 4a
- C2 -: Breite von 4b
- C3 -: Breite von 4c
- D -: Länge von 7b

## Patentansprüche

1. Katheter mit einem zur teilweise intrakorporalen Verwendung vorgesehenen Katheterabschnitt (2), **dadurch gekennzeichnet, dass** der Katheterabschnitt (2) durch eine Platte (4a, 4b, 4c) hindurch geführt ist, wobei zumindest die Oberfläche der Platte (4a, 4b, 4c) eine antimikrobiell wirksame Metall-Legierung aufweist.

2. Katheter nach Anspruch 1, **dadurch gekennzeichnet, dass** der Katheterabschnitt (2) einen Durchmesser (B) aufweist und die Platte (4a, 4b, 4c) eine Breite (C1, C2, C3) aufweist, wobei der Durchmesser (B) und die Breite (C1, C2, C3) mindestens ein Verhältnis von 1 : 2 zueinander aufweisen.

3. Katheter nach Anspruch 1 oder 2, **dadurch gekennzeichnet, dass** die Platte (4a, 4b, 4c) eine Dicke (A) aufweist, wobei die Dicke (A) und die Breite (C1, C2, C3) der Platte (4a, 4b, 4c) mindestens ein Verhältnis von 1 : 5 zueinander aufweisen.

4. Katheter nach einem der Ansprüche 1 bis 3, **dadurch gekennzeichnet, dass** die Platte (4a, 4b, 4c) flach oder kalottenförmig ausgebildet ist.

5. Katheter nach einem der Ansprüche 1 bis 4, **dadurch gekennzeichnet, dass** der Katheterabschnitt (2) zumindest bereichsweise von einem Röhrchen (6) umgeben ist, wobei zumindest die Oberfläche des Röhrchens (6) eine antimikrobielle Metall-Legierung aufweist.

6. Katheter nach einem der Ansprüche 1 bis 5, **dadurch gekennzeichnet, dass** die Metall-Legierung eine Cu-Legierung ist, welche mehr als 60 Gew.-% Kupfer aufweist.

7. Katheter nach einem der Ansprüche 1 bis 6, **dadurch gekennzeichnet, dass** die Metall-Legierung jeweils maximal 0,01 Gew.-% Nickel und/oder Blei aufweist.

8. Katheter nach einem der Ansprüche 1 bis 7, **dadurch gekennzeichnet, dass** die Platte (4a, 4b, 4c) und/oder das Röhrchen (6) gegenüber dem Katheterabschnitt (2) relativ verschieblich sind/ist.

9. Katheter nach einem der Ansprüche 1 bis 8, **dadurch gekennzeichnet, dass** die Platte (4a, 4b, 4c) im eingeführten Zustand des Katheterabschnitts (2) auf einem umliegenden Hautbereich (5) durch ein Mittel zur Lagefixierung extrakorporal festgelegt ist.

10. Katheter nach einem der Ansprüche 1 bis 9, **dadurch gekennzeichnet, dass** das Mittel zur Lagefixierung einzelne Fäden aus einer antimikrobiellen Metall-Legierung aufweist, wobei die Platte (4a, 4b, 4c) und/oder das Röhrchen (6) mit dem Mittel zur Lagefixierung zumindest bereichsweise umwickelt sind/ist.
